# EUROPEAN PATENT APPLICATION

(11) **EP 4 696 250 A1**
(43) Date of publication of application: **18.02.2026**
(21) Application number: 25740660.3
(22) Date of filing: 06.06.2025
(51) Int. Cl.: A61B 17/072, A61B 17/068, A61B 17/11

(54) **STAPLE MAGAZINE ASSEMBLY AND STAPLER**

(30) Priority: 07.06.2024 CN 202421299970 U
(71) Applicant: Ningbo Verykind Medical Device Co., Ltd., Ningbo, Zhejiang 315000 (CN)
(72) Inventor: CHEN, Zaihong, Ningbo, Zhejiang 315000 (CN); SHAO, Dingding, Ningbo, Zhejiang 315000 (CN)
(74) Representative: Gamba, Alessandro
(86) International application number: PCT/CN2025/099701
(87) International publication number: WO 2025/252224

(57) **Abstract**

The embodiments of the present invention provide a staple cartridge assembly and an anastomat, relating to the technical field of surgical instruments. The staple cartridge assembly includes a staple cartridge and an elastic limiting unit, wherein a mounting hole is provided on the staple cartridge, and the elastic limiting unit is arranged at the mounting hole; the elastic limiting unit comprises an elastic force-transmitting structure, and a trigger portion and a limiting portion that are located at two ends of the elastic force-transmitting structure, wherein the trigger portion is close to an inner end of the mounting hole, and the limiting portion is close to an outer end of the mounting hole, the trigger portion is configured for being arranged on a movement path of a staple pushing member moving along its staple pushing direction, the limiting portion is provided corresponding to a setting region of an outer surface of the staple cartridge, and the setting region is a region of the outer surface of the staple cartridge configured to face the staple cartridge base. In the embodiments of the present invention, the difficulty of mounting the staple cartridge assembly in which at least a portion of the staples has been fired into the staple cartridge base will be greatly increased, which can prevent incorrect mounting of the staple cartridge assembly in which at least a portion of the staples has been fired. The anastomat includes the above-mentioned staple cartridge assembly.

## Description

### CROSS-REFERENCE TO RELATED APPLICATIONS

The present invention claims the priority to the Chinese patent application with the filing No. 202421299970.1 filed with the Chinese Patent Office on June 07, 2024, and entitled "STAPLE CARTRIDGE ASSEMBLY AND ANASTOMAT", the contents of which are incorporated herein by reference in entirety.

### TECHNICAL FIELD

The present invention relates to the technical field of surgical instruments, and particularly to a staple cartridge assembly and an anastomat.

### BACKGROUND ART

The anastomat is a medical instrument commonly used for tissue anastomosis and suturing in surgical operations. It comprises components such as a staple cartridge base, a staple cartridge assembly, and an anvil. The staple cartridge assembly is usually detachably connected with the staple cartridge base, for example, mounted in a slot-shaped structure of the staple cartridge base. During a surgical operation, a pushing structure, such as a cutting knife, pushes a staple pushing member to move, and through the staple pushing movement of the staple pushing member, anastomotic staples are fired. The fired anastomotic staples contact the staple forming grooves on the anvil and deform, for example, are formed into a B-shape, thereby completing the anastomosis operation.

It is observed that the staple cartridge assembly provides a risk that the anastomotic staples are fired and reloaded into the staple cartridge base, which may, in minor cases, delay the medical procedure, or, in severe cases, cause medical accidents.

### SUMMARY

The objective of the present invention includes, for example, providing a staple cartridge assembly and an anastomat, so as to improve deficiencies in the related technology.

The embodiments of the present invention can be implemented as follows.

The embodiment of the present invention provides a staple cartridge assembly, comprising a staple cartridge and an elastic limiting unit. A mounting hole is provided on the staple cartridge, and the elastic limiting unit is arranged at the mounting hole. The elastic limiting unit comprises an elastic force-transmitting structure, and a trigger portion and a limiting portion that are located at two ends of the elastic force-transmitting structure, wherein the trigger portion is close to an inner end of the mounting hole, and the limiting portion is close to an outer end of the mounting hole. The trigger portion is configured to be arranged on a movement path of a staple pushing member moving along its staple pushing direction. The limiting portion is provided corresponding to a setting region of an outer surface of the staple cartridge, and the setting region is a region of the outer surface of the staple cartridge configured to face the staple cartridge base.

Optionally, the staple cartridge is correspondingly provided with the trigger portion at an end limiting position where the staple pushing member moves along its staple pushing direction.

Optionally, the elastic limiting unit is an elastic piece structure, a fixed end of the elastic piece structure is fixed to the mounting hole, and the elastic force-transmitting structure, the trigger portion, and the limiting portion are all formed on the elastic piece structure.

Optionally, the elastic piece structure is integrally molded, or the elastic piece structure is formed by connecting multiple elastic pieces.

The elastic piece structure is bent to form a first section, a second section, and a third section that are sequentially connected. The first section is on a circumferential side of the mounting hole and is fixedly connected with the staple cartridge, so as to form a fixed end of the spring tab structure. The second section and the third section are respectively arranged obliquely relative to the first section, the third section is arranged at an angle with the second section, and the opening direction of the angle formed by the second section and the third section is consistent with a depth direction of the mounting hole. The third section forms the elastic force-transmitting structure.

Optionally, the first section is connected with an insertion section, the staple cartridge is provided with an insertion slot, and the insertion section is inserted into the insertion slot.

Optionally, an inner surface of the mounting hole is arranged as an inclined surface, and the inclined surface is in contact with the second section or the third section.

Optionally, the elastic limiting unit comprises at least one trigger portion and one limiting portion.

Optionally, the staple cartridge base is provided with a first slot-shaped structure, the staple cartridge assembly is mounted inside the first slot-shaped structure, and at least a top end of the staple cartridge assembly is exposed outside the first slot-shaped structure.

Optionally, the staple cartridge base is provided with a first slot-shaped structure, and the staple cartridge assembly is placed into the first slot-shaped structure along a setting direction. The first section, the second section, and the third section are sequentially connected along the setting direction.

Optionally, the staple cartridge assembly further comprises a staple cartridge cover, the staple cartridge cover is detachably connected with the staple cartridge, and the staple cartridge assembly is connected with the staple cartridge base through the staple cartridge cover.

Optionally, a through hole is provided on the staple cartridge cover, the through hole is in communication with the mounting hole, and the through hole is configured to allow the limiting portion to pass through.

Optionally, the staple cartridge assembly further comprises a staple cartridge cover, the staple cartridge cover is detachably connected with the staple cartridge, and the staple cartridge assembly is connected with the staple cartridge base through the staple cartridge cover. A through hole is provided on the staple cartridge cover, the through hole is in communication with the mounting hole, and the through hole is configured to allow the limiting portion to pass through. The staple cartridge cover tightly presses the fixed end of the elastic piece structure against an end surface at the outer end of the mounting hole.

Optionally, first side walls on both sides in a width direction of the staple cartridge are both provided with the mounting holes;
and/or, the trigger portion and the limiting portion are both configured as sliding members, the sliding members are slidably mounted on the staple cartridge along a depth direction of the mounting hole, the elastic force-transmitting structure is arranged inside the mounting hole, and two ends of the elastic force-transmitting structure are respectively in contact with the two sliding members.

Optionally, the trigger portion is provided with a guide surface, and/or the limiting portion is provided with a guide surface.

The present invention further provides an anastomat, which comprises the above staple cartridge assembly.

Optionally, the anastomat further comprises a staple cartridge base and an anvil, the staple cartridge base is detachably connected with the staple cartridge assembly, and the anvil is arranged at a top end of the staple cartridge assembly.

Optionally, a first forming groove is provided on the anvil, and a fired staple strikes the first forming groove and undergoes deformation.

In combination with the above technical solutions, the beneficial effects of the embodiments of the present invention include the following.

In the staple cartridge assembly of the present invention, a mounting hole is provided on the staple cartridge, and an elastic limiting unit is mounted at the mounting hole to realize the mounting of the elastic limiting unit on the staple cartridge. The elastic limiting unit includes an elastic force-transmitting structure, and a trigger portion and a limiting portion that are located at two ends of the elastic force-transmitting structure, wherein the trigger portion is close to an inner end of the mounting hole, and the limiting portion is close to an outer end of the mounting hole. When the staple cartridge assembly is mounted on the staple cartridge base of the anastomat, the limiting portion is provided corresponding to a setting region of an outer surface of the staple cartridge. The setting region is a region of the outer surface of the staple cartridge configured to face the staple cartridge base, for example, arranged opposite to an inner wall of a first slot-shaped structure provided on the staple cartridge base for mounting the staple cartridge assembly. When the staple pushing member moves along its staple pushing direction, for example from rear to front, and contacts the trigger portion, at least some of the staples in the staple cartridge are fired. The trigger portion, under interaction with the staple pushing member, moves outward from the staple cartridge. The elastic force-transmitting structure, under the action of the trigger portion, drives the limiting portion to move outward from the staple cartridge, such that the limiting portion abuts against the inner wall of the first slot-shaped structure of the staple cartridge base. At this moment, the elastic force-transmitting structure stores relatively high potential energy, but it does not affect the normal use of the staple cartridge assembly in the staple cartridge base, and the staple cartridge assembly can be normally removed from the staple cartridge base. If the staple cartridge assembly is removed from the staple cartridge base, the abutment effect of the staple cartridge base on the limiting portion is released. Under the potential energy of the elastic force-transmitting structure, the limiting portion moves outward from the staple cartridge, thereby increasing the dimension of the staple cartridge assembly at the location of the limiting portion. This essentially forms a stop, specifically, a stop that obstructs the remounting of the staple cartridge assembly into the staple cartridge base in this state. In other words, the difficulty of mounting the staple cartridge assembly, in which at least some of the staples have been fired, into the staple cartridge base will be greatly increased, thereby preventing, to a certain extent, incorrect mounting of a staple cartridge assembly in which at least some of the staples have been fired.

The anastomat of the present invention comprises the above-mentioned staple cartridge assembly and possesses all the beneficial effects of the staple cartridge assembly.

### BRIEF DESCRIPTION OF DRAWINGS

In order to more clearly illustrate the specific embodiments of the present invention or the technical solution in the prior art, the drawings required to be used in the description of the specific embodiment or the prior art will be briefly introduced as follows. Obviously, the drawings described below are some embodiments of the present invention. Those of ordinary skill in the art, without paying inventive labor, may also obtain other drawings according to these drawings.
FIG. 1 is an exploded schematic view of a staple cartridge assembly in an embodiment of the present invention;
FIG. 2 is a schematic perspective view of a staple cartridge assembly in which an upper portion of the staple cartridge is removed to expose an elastic limiting unit in an embodiment of the present invention;
FIG. 3 is a schematic perspective view showing a staple cartridge assembly mounted in a first slot-shaped structure of a staple cartridge base in an embodiment of the present invention;
FIG. 4 is a schematic structural view of an anastomat from another viewing angle corresponding to FIG. 3;
FIG. 5 is an exploded schematic view of an anastomat when a staple pushing member is about to contact a trigger portion of an elastic limiting unit in an embodiment of the present invention; and
FIG. 6 is a schematic view showing a limiting portion of an elastic limiting unit snapped in a staple cartridge base in an embodiment of the present invention.

1 - staple cartridge assembly; 11 - staple cartridge; 111 - mounting hole; 112 - first side wall; 113 - insertion slot; 114 - setting region; 12 - elastic limiting unit; 121 - trigger portion; 122 - limiting portion; 123 - elastic force-transmitting structure; 12a - first section; 12b - second section; 12c - third section; 12d - insertion section; 13 - staple pushing member; 14 - staple cartridge cover; 141 - through hole; 2 - staple cartridge base; 21 - first slot-shaped structure; 3 - anvil.

### DETAILED DESCRIPTION OF EMBODIMENTS

In order to make the above objectives, features, and advantages of the present invention more apparent and understandable, specific embodiments of the present invention are described in detail below in conjunction with the drawings.

In the description of the present invention, it should be noted that, unless otherwise clearly stipulated and limited, if terms "mount", "interconnect", "connect", and "connect" appear, they should be understood in a broad sense. For example, it can be a fixed connection, a detachable connection, or an integral connection; and it can be a direct connection, an indirect connection through an intermediary. Those of ordinary skill in the art can understand the meanings of the above terms in the present invention according to specific situations.

In the description of the present invention, if reference terms such as "embodiment", "one embodiment", "some embodiments", "exemplarily", and "one mode of embodiment" appear, such descriptions mean that the specific features, structures, materials, or characteristics described in connection with the embodiment or mode of embodiment are included in at least one embodiment or example of the present invention. The indicative expressions of the above terms in the specification do not necessarily refer to the same embodiment or example. Moreover, the specific features, structures, materials, or characteristics described can be combined in a suitable manner in any one or more embodiments or examples.

Additionally, the terms "first" and "second" are used for descriptive objectives only and should not be understood as indicating or implying relative importance or specifying the quantity of the indicated technical features. Thus, features defined with "first" and "second" may expressly or implicitly include at least one of such features.

In the drawings of the present invention, the Z-axis indicates the vertical direction, that is, the up-and-down position, and the positive direction of the Z-axis (that is, the direction pointed by the arrow of the Z-axis) indicates up, and the negative direction of the Z-axis indicates down. The X-axis in the drawings indicates the front-and-back position, and the positive direction of the X-axis (that is, the direction pointed by the arrow of the X-axis) indicates the front side, and the negative direction of the X-axis indicates the rear side. The Y-axis in the drawings indicates the horizontal direction and is designated as the left-and-right position, and the positive direction of the Y-axis (that is, the direction pointed by the arrow of the Y-axis) indicates the right side, and the negative direction of the Y-axis indicates the left side. Meanwhile, it should be stated that the meaning of the representations of the Z-axis, Y-axis, and X-axis above is only for facilitating the description of the present invention and simplifying the description, and is not intended to indicate or imply that the referred device or element must have a specific orientation or be constructed and operated in a specific orientation, therefore it should not be understood as a limitation to the present invention.

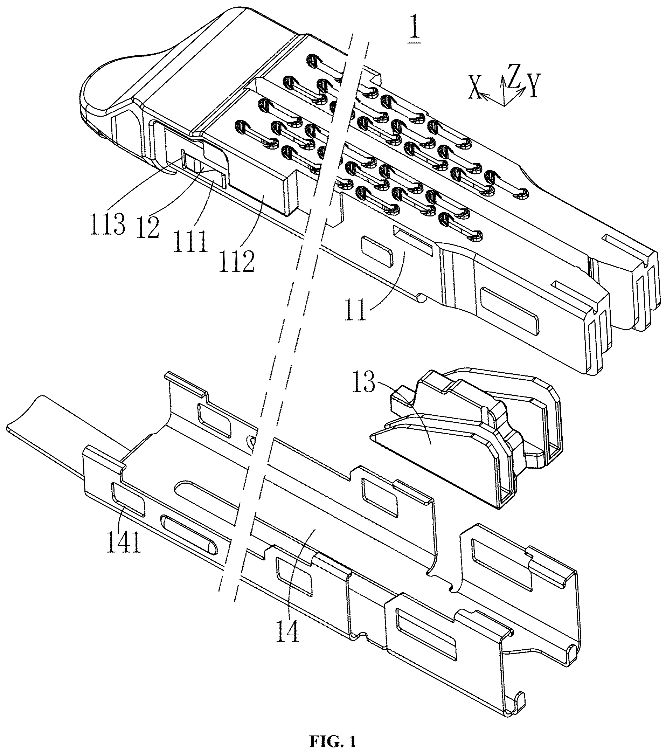

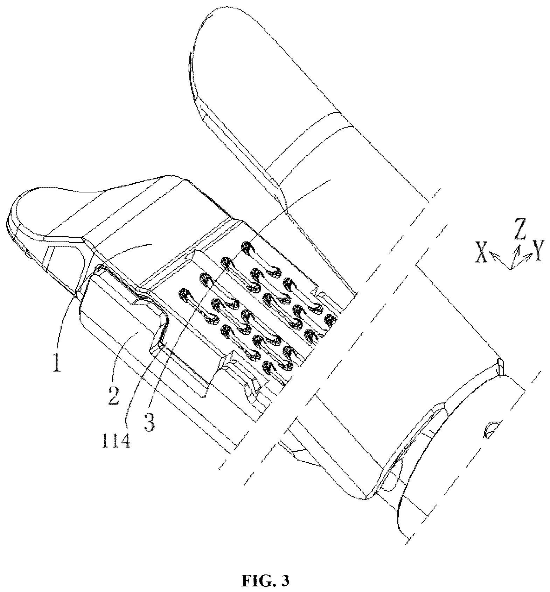

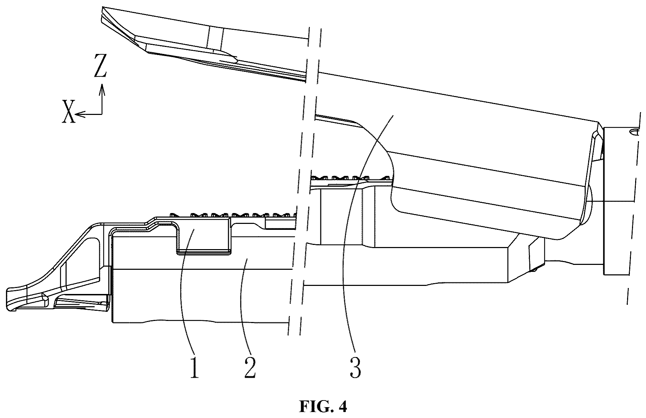

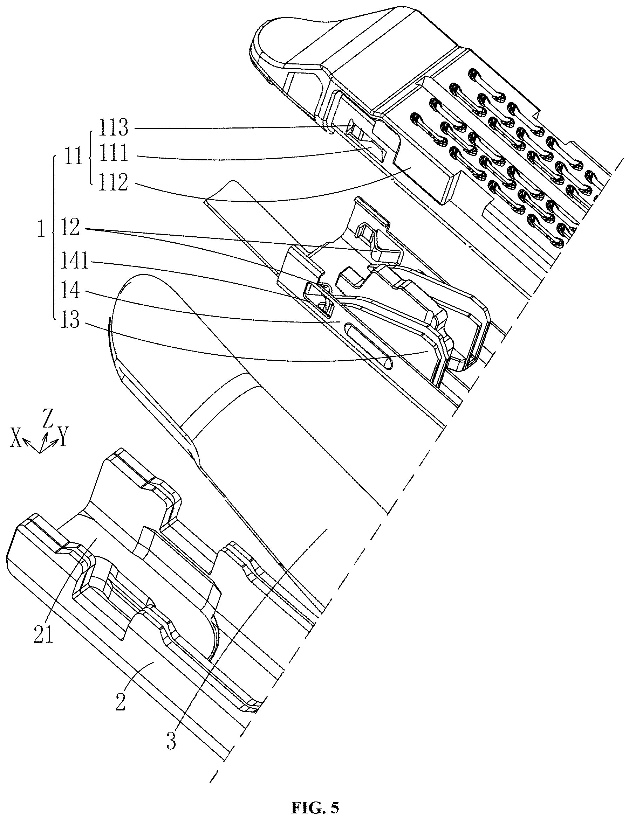

As shown in FIG. 1 to FIG. 6, an embodiment of the present invention provides a staple cartridge assembly 1, comprising a staple cartridge 11 and an elastic limiting unit 12, a mounting hole 111 is provided on the staple cartridge 11, and the elastic limiting unit 12 is arranged at the mounting hole 111. The elastic limiting unit 12 comprises an elastic force-transmitting structure 123, and a trigger portion 121 and a limiting portion 122 that are located at two ends of the elastic force-transmitting structure 123. The trigger portion 121 is close to the inner end of the mounting hole 111, and the limiting portion 122 is close to the outer end of the mounting hole 111. The trigger portion 121 is configured to be arranged on the movement path of a staple pushing member 13 moving along its staple pushing direction. The limiting portion 122 is arranged in a setting region 114, wherein the setting region 114 is a region where the staple cartridge 11 and the staple cartridge base 2 are arranged spaced apart and opposite to each other, and the setting region 114 is located outside the staple cartridge 11.

In the present invention, the staple cartridge assembly 1 can be applied to an anastomat, the anastomat comprises a staple cartridge base 2 and an anvil 3 that are located at a front end, and the anvil 3 and the staple cartridge base 2 can move relative to each other to realize opening and closing.

The staple cartridge assembly 1 is mounted on the staple cartridge base 2. For example, the staple cartridge base 2 is provided with a first slot-shaped structure 21, the staple cartridge assembly 1 is mounted inside the first slot-shaped structure 21, and at least a top end of the staple cartridge assembly 1 is exposed outside the first slot-shaped structure 21. Therefore, subsequently, the anastomotic staples can be pushed out from the top end of the staple cartridge assembly 1, and the pushed-out anastomotic staples contact the anvil 3.

The staple pushing direction of the staple pushing member 13 is from rear to front. During actual use, the anastomat is inserted into a preset operation position, and the anvil 3 and the staple cartridge base 2 are closed and press the tissue to be sutured tightly. Under the action of a cutting knife, for example, the staple pushing member 13 moves from rear to front, driving the anastomotic staples to be fired sequentially from rear to front, for example, fired upward. The fired anastomotic staples strike the first forming grooves (not shown in the figure) on the anvil 3, and the anastomotic staples are deformed to suture the tissue to be sutured. For example, the anastomotic staples can be deformed into a B-shape.

It should be noted that the initial limit position of the staple pushing member 13 is located at the rear end of the staple cartridge 11. The staple pushing member 13 is spaced apart from the trigger portion 121, and the limiting portion 122 maintains its original state and does not move toward the outside of the staple cartridge 11. That is, the limiting portion 122 is spaced apart from the staple cartridge base 2, and the staple cartridge assembly 1 can be smoothly loaded into the first slot-shaped structure 21 of the staple cartridge base 2.

In the present invention, the mounting hole 111 can be provided on a first side wall 112 of the staple cartridge 11, and the mounting hole 111 can also be provided on a bottom plate of the staple cartridge 11. This is not limited.

The trigger portion 121 of the elastic limiting unit 12 is close to the inner end of the mounting hole 111. For example, the trigger portion 121 is arranged inside the mounting hole 111 and away from the outer end of the mounting hole 111. The trigger portion 121 is located on the staple-pushing movement path of the staple pushing member 13. The limiting portion 122 of the elastic limiting unit 12 is close to the outer end of the mounting hole 111. For example, the limiting portion 122 is arranged in the setting region 114.

The specific structure of the elastic limiting unit 12 is not limited, as long as it includes the elastic force-transmitting structure 123, and the trigger portion 121 and the limiting portion 122 that are located at two ends of the elastic force-transmitting structure 123. The elastic force-transmitting structure 123 connects the trigger portion 121 and the limiting portion 122. The elastic force-transmitting structure 123 transmits the acting force between the trigger portion 121 and the limiting portion 122. For example, when the limiting portion 122 is not blocked in the left-and-right direction and the trigger portion 121 is pressed in the left-and-right direction, the elastic force-transmitting structure 123 can transmit the pressure to the limiting portion 122 and make the limiting portion 122 move; alternatively, when the trigger portion 121 is not blocked in the left-and-right direction and the limiting portion 122 is pressed in the left-and-right direction, the elastic force-transmitting structure 123 can transmit the pressure to the trigger portion 121 and make the trigger portion 121 move. For another example, when the limiting portion 122 is blocked in the left-and-right direction and the trigger portion 121 is pressed in the left-and-right direction, the elastic force-transmitting structure 123 deforms to accumulate energy in the form of potential energy and reduces the distance between the limiting portion 122 and the trigger portion 121 in the left-and-right direction; alternatively, when the trigger portion 121 is blocked in the left-and-right direction and the limiting portion 122 is pressed in the left-and-right direction, the elastic force-transmitting structure 123 deforms to accumulate energy in the form of potential energy and reduces the distance between the limiting portion 122 and the trigger portion 121 in the left-and-right direction.

When the staple pushing member 13 is pushed so that the staple pushing member 13 moves along its staple-pushing path, for example, moves from rear to front to contact with the trigger portion 121, at least some of the anastomotic staples in the staple cartridge 11 are fired, the trigger portion 121 moves toward the outside of the staple cartridge 11 under the pushing action of the staple pushing member 13, the elastic force-transmitting structure 123 drives the limiting portion 122 to move toward the outside of the staple cartridge 11, and the limiting portion 122 abuts against the inner wall of the first slot-shaped structure 21. At this time, the staple cartridge assembly 1 can be normally disassembled from the staple cartridge base 2.

When the staple cartridge assembly 1 is disassembled from the staple cartridge base 2, the resisting effect of the staple cartridge base 2 on the limiting portion 122 is released. Under the potential energy action of the elastic force-transmitting structure 123, the limiting portion 122 moves toward the outside of the staple cartridge 11, so that the size of the staple cartridge assembly 1 increases, forming a blocking state. Specifically, in the blocking state, the staple cartridge assembly 1 cannot be re-mounted into the staple cartridge base 2, thereby preventing the staple cartridge assembly 1 from being incorrectly mounted.

Optionally, the trigger portion 121 is arranged at the end limiting position of the staple-pushing path of the staple pushing member 13.

The staple cartridge assembly 1 of the present embodiment includes only one elastic limiting unit 12, the elastic limiting unit 12 includes only one trigger portion 121 and one limiting portion 122, and the position of the mounting hole 111 corresponds to the end limiting position of the staple pushing member 13.

However, it should be understood that multiple mounting holes 111 can also be arranged, and multiple elastic limiting units 12 can be correspondingly arranged. Alternatively, the elastic limiting unit 12 can include multiple trigger portions 121, multiple elastic force-transmitting structures 123, and multiple limiting portions 122. When the staple pushing member 13 completes the firing of different quantities of anastomotic staples and the staple pushing member 13 is located at different positions, the corresponding trigger portion 121 at the corresponding position can be triggered to make the corresponding limiting portion 122 move, so as to prevent incorrect mounting operation of the disassembled staple cartridge assembly 1.

It should be understood that the end limiting position of the staple pushing member 13 is the position where the staple pushing member 13 is located at the foremost end of the staple cartridge 11. When the staple pushing member 13 is in the state of the end limiting position, all anastomotic staples in the staple cartridge 11 have already been fired, that is, the staple cartridge 11 is in an empty state.

After the staple cartridge assembly 1 is disassembled from the staple cartridge base 2, the limiting portion 122 of the elastic limiting unit 12 extends out from the mounting hole 111, so as to prevent the already empty staple cartridge assembly 1 from being incorrectly mounted, and to avoid assembling the empty staple cartridge assembly 1 into the staple cartridge base 2, thereby avoiding adverse effects.

Optionally, the staple cartridge assembly 1 further comprises a staple cartridge cover 14, the staple cartridge cover 14 is detachably connected with the staple cartridge 11, and the staple cartridge cover 14 is connected with the staple cartridge base 2. A through hole 141 is provided on the staple cartridge cover 14, the through hole 141 is in communication with the mounting hole 111, and the through hole 141 is configured to allow the limiting portion 122 to pass through.

The detachable connection between the staple cartridge cover 14 and the staple cartridge 11 can be a snap-fit connection. The staple cartridge assembly 1 is connected with the first slot-shaped structure 21 of the staple cartridge base 2 through the staple cartridge cover 14, for example, the connection is a snap-fit connection. The specific connection manner between the two can adopt related technologies, for example, related technologies of the applicant of the present invention before the application date, which will not be described in detail here.

The staple cartridge cover 14 can protect the bottom of the staple cartridge 11, and also enables the limiting portion 122 to smoothly move toward the outside of the staple cartridge 11, for example, move in the left-and-right direction and abut against the inner wall of the first slot-shaped structure 21.

Optionally, the elastic limiting unit 12 is an elastic piece structure, a fixed end of the elastic piece structure is fixed to the mounting hole 111, and the elastic piece structure further comprises a limiting portion 122, an elastic force-transmitting structure 123, and a trigger portion 121, which are sequentially connected. The limiting portion 122 is arranged in the mounting hole 111. The elastic limiting unit 12 has a simple structure and high reliability.

Specifically, the elastic piece structure can be formed by one elastic piece or by connecting multiple elastic pieces. This is not limited, as long as it meets usage requirements.

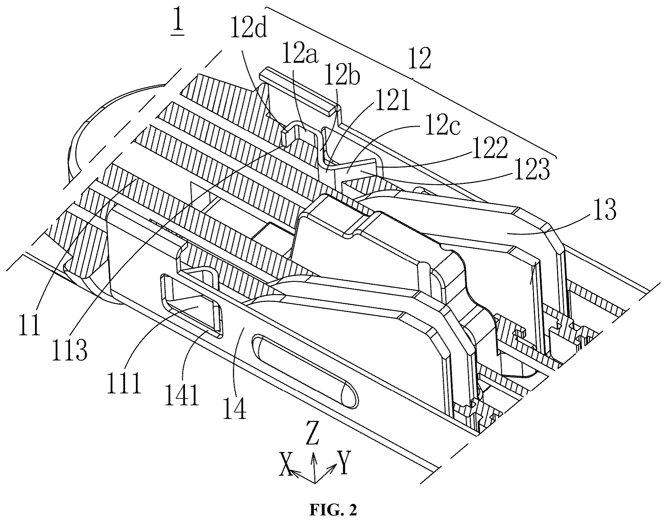

As shown in FIG. 2, optionally, the elastic piece structure is bent to form a first section 12a, a second section 12b, and a third section 12c that are sequentially connected. The first section 12a is on a circumferential side of the mounting hole 111 and is fixedly connected with the staple cartridge 11, so as to form a fixed end of the spring tab structure. The second section 12b and the third section 12c are respectively arranged obliquely relative to the first section 12a, the third section 12c is arranged at an angle with the second section 12b, and the opening direction of the angle formed by the second section 12b and the third section 12c faces the mounting hole 111. The third section 12c forms the elastic force-transmitting structure 123.

As shown in FIG. 2, optionally, the elastic piece structure fits against the staple cartridge 11 at the first section 12a, so as to fix the fixed end of the elastic piece structure to the staple cartridge 11. The limiting portion 122 is arranged on the third section 12c and close to one end near the outside of the mounting hole 111. The trigger portion 121 is arranged on the third section 12c and close to one end near the inside of the mounting hole 111. The second section 12b is arranged obliquely relative to the first section 12a.

The elastic limiting unit 12 can be formed by bending a single elastic piece, without needing to form the elastic limiting unit 12 using multiple components. This enables a simple structure, so that the third section 12c as a whole can move relative to the first section 12a, and is reset by the second section 12b. For example, one end of the third section 12c, close to the second section 12b, can perform resettable movement relative to the first section 12a in the depth direction of the mounting hole 111, that is, in the left-and-right direction. When the third section 12c is arranged obliquely relative to the first section 12a, the trigger portion 121 and the limiting portion 122 of the third section 12c can move relative to each other, and potential energy can be accumulated to a certain extent through deformation of the third section 12c. The relative position of the trigger portion 121 and the limiting portion 122 can be reset through deformation of the third section 12c itself, which enables a simple structure and strong practicality.

Optionally, the first section 12a is connected with an insertion section 12d, and an insertion slot 113 is provided on the staple cartridge 11. The insertion section 12d is inserted into the insertion slot 113.

Optionally, one end of the first section 12a away from the second section 12b is bent to form the insertion portion, and the depth direction of the insertion slot 113 can be consistent with the depth direction of the mounting hole 111. As shown in FIG. 1, FIG. 2, and FIG. 5, the first section 12a is at the outer end of the mounting hole 111 and fits against the staple cartridge 11. One end of the first section 12a, away from the second section 12b, is bent inward toward the staple cartridge 11 to form the insertion portion.

Through the insertion connection between the insertion section 12d and the insertion slot 113, the reliability of the connection between the fixed end of the elastic piece structure and the staple cartridge 11 can be improved.

It should be understood that one or more of the trigger portion 121 and the limiting portion 122 can be provided with a guide surface as needed. The guide surface is configured to contact for guiding. For example, one or more of the trigger portion 121 and the limiting portion 122 can be configured as a spherical convex structure, so that when the staple pushing member 13 contacts the trigger portion 121, scratching between the two can be avoided, thereby preventing damage to the contact surface. When the limiting portion 122 contacts the staple cartridge base 2, scratching between the two can be avoided, thereby preventing contact surface damage, improving the service life of the staple pushing member 13 and the elastic limiting unit 12, and reducing abnormal noise and the possibility of debris caused by scratching.

In the embodiment, the staple cartridge base 2 is provided with a first slot-shaped structure 21. The staple cartridge assembly 1 can be placed into the first slot-shaped structure 21 along a setting direction. The first section 12a, the second section 12b, and the third section 12c are sequentially connected along the setting direction.

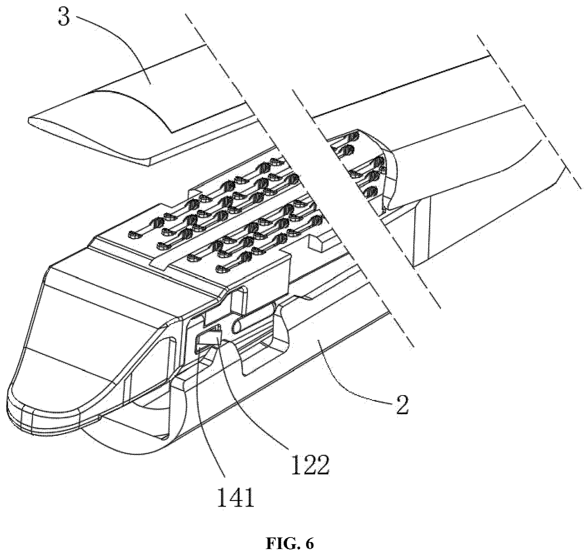

The setting direction is from front to rear. Specifically, the staple cartridge assembly 1 moves from front to rear to be placed into the first slot-shaped structure 21. The first section 12a, the second section 12b, and the third section 12c are sequentially distributed from front to rear. As shown in FIG. 2 and FIG. 6, one end of the third section 12c forms the limiting portion 122, and the other end forms the trigger portion 121. When the staple cartridge assembly 1 needs to be pushed into the first slot-shaped structure 21 from front to rear, the limiting portion 122 at the end of the third section 12c protrudes in the left-and-right direction beyond the outer surface of the staple cartridge assembly 1, for example, beyond the outer surface of the staple cartridge cover 14. The front-end surface of the first slot-shaped structure 21 of the staple cartridge base 2 will abut against the limiting portion 122. As the staple cartridge assembly 1 is pushed from front to rear, the limiting portion 122 tends to be stressed and move forward, and at least initially has a tendency to expand outward. Therefore, the difficulty of pushing the staple cartridge assembly 1 from front to rear into the first slot-shaped structure 21 is relatively high, thereby providing a better effect of preventing incorrect mounting.

In the present embodiment, optionally, the staple cartridge cover 14 tightly presses the fixed end of the elastic piece structure against the end surface at the outer end of the mounting hole 111.

As shown in FIG. 2, the inner surface of the staple cartridge cover 14 in the left-and-right direction presses the first section 12a against the end surface at the outer end of the mounting hole 111, and keeps the insertion section 12d inserted into the insertion slot 113, thereby ensuring the reliability of the connection between the elastic piece structure and the staple cartridge 11.

It should be understood that a receiving groove can be provided on the end surface at the outer end of the mounting hole 111 of the staple cartridge 11. The receiving groove can accommodate the elastic piece structure, for example, a receiving groove for accommodating the first section 12a.

Optionally, along the direction in which the first section 12a, the second section 12b, and the third section 12c are sequentially distributed, the inner surface of the mounting hole 111 can be arranged as an inclined surface, and the inclined surface is used to contact the second section 12b or the third section 12c to limit its terminal position.

In the present embodiment, optionally, the first side wall 112 of the staple cartridge 11 in the width direction is provided with mounting holes 111. For example, two mounting holes 111 are symmetrically provided in the left-and-right direction. During mis-mounting prevention, both left and right sides of the staple cartridge assembly 1 are blocked by the two limiting portions 122, so that the force is balanced, and damage to the staple cartridge assembly 1 caused by unbalanced force on the left and right sides is avoided, thus providing high reliability.

It should be understood that the above description of the structure of the elastic limiting unit 12 is exemplary and is not limited thereto.

Optionally, in the solution of the elastic piece structure, one end of the second section 12b away from the first section 12a can be connected between the front and rear ends of the third section 12c.

Optionally, the trigger portion 121 and the limiting portion 122 can both be sliding members. The sliding members can slide in the depth direction of the mounting hole 111 (for example, the left-and-right direction) to be mounted on the staple cartridge 11. The elastic force-transmitting structure 123 (for example, an elastic piece or a compression spring) is located between the sliding member where the trigger portion 121 is located and the sliding member where the limiting portion 122 is located. The acting force between the two is transmitted by the elastic force-transmitting structure 123. The sliding terminal positions of the trigger portion 121 and the limiting portion 122 should both be limited.

An embodiment of the present invention further provides an anastomat, the anastomat comprises the above-mentioned staple cartridge assembly 1, and the anastomat can be applied in suturing tissues in abdominal surgery, thoracic surgery, and the like. The application scope of the anastomat is not limited. The anastomat has all the beneficial effects of the staple cartridge assembly 1.

### INDUSTRIAL PRACTICALITY

In conclusion, the present invention provides a staple cartridge assembly and an anastomat. The overall structure is simple, the cost is low, and it also prevents a staple cartridge assembly in which the anastomotic staples have been partially fired from being incorrectly mounted.

Although the present invention is disclosed as above, the protection scope of the present invention is not limited thereto. Those skilled in the art may make various changes and modifications without departing from the spirit and scope of the present invention. These changes and modifications shall fall within the protection scope of the present invention.

## Claims

1. A staple cartridge assembly, **characterized by** comprising a staple cartridge (11) and an elastic limiting unit (12), wherein a mounting hole (111) is provided on the staple cartridge (11), and the elastic limiting unit (12) is arranged at the mounting hole (111); the elastic limiting unit (12) comprises an elastic force-transmitting structure (123), and a trigger portion (121) and a limiting portion (122) that are located at two ends of the elastic force-transmitting structure (123), wherein the trigger portion (121) is close to an inner end of the mounting hole (111), and the limiting portion (122) is close to an outer end of the mounting hole (111), the trigger portion (121) is configured for being arranged on a movement path of a staple pushing member (13) moving along its staple pushing direction, the limiting portion (122) is provided corresponding to a setting region of an outer surface of the staple cartridge (11), and the setting region is a region of the outer surface of the staple cartridge (11) configured to face the staple cartridge base (2).

2. The staple cartridge assembly according to claim 1, wherein the staple cartridge (11) is correspondingly provided with the trigger portion (121) at an end limiting position where the staple pushing member (13) moves along its staple pushing direction.

3. The staple cartridge assembly according to claim 1 or 2, wherein the elastic limiting unit (12) is an elastic piece structure, a fixed end of the elastic piece structure is fixed to the mounting hole (111), and the elastic force-transmitting structure (123), the trigger portion (121), and the limiting portion (122) are all formed on the elastic piece structure.

4. The staple cartridge assembly according to claim 3, wherein the elastic piece structure is integrally molded, or the elastic piece structure is formed by connecting multiple elastic pieces.

5. The staple cartridge assembly according to claim 3 or 4, wherein the elastic piece structure is bent to form a first section (12a), a second section (12b), and a third section (12c) which are sequentially connected, the first section (12a) is located on a circumferential side of the mounting hole (111) and is fixedly connected with the staple cartridge (11) to form the fixed end of the elastic piece structure; and
the second section (12b) and the third section (12c) are respectively arranged obliquely relative to the first section (12a), the third section (12c) is arranged at an angle with the second section (12b), an opening direction of the angle formed by the second section (12b) and the third section (12c) is consistent with a depth direction of the mounting hole (111), and the third section (12c) forms the elastic force-transmitting structure (123).

6. The staple cartridge assembly according to claim 5, wherein the first section (12a) is connected with an insertion section (12d), and the staple cartridge (11) is provided with an insertion slot (113), and the insertion section (12d) is inserted into the insertion slot (113).

7. The staple cartridge assembly according to claim 5 or 6, wherein an inner surface of the mounting hole (111) is arranged as an inclined surface, and the inclined surface is in contact with the second section (12b) or the third section (12c).

8. The staple cartridge assembly according to any one of claims 1 to 7, wherein the elastic limiting unit (12) comprises at least one trigger portion (121) and one limiting portion (122).

9. The staple cartridge assembly according to any one of claims 1 to 8, wherein the staple cartridge base (2) is provided with a first slot-shaped structure (21), the staple cartridge assembly is mounted inside the first slot-shaped structure (21), and at least a top end of the staple cartridge assembly is exposed outside the first slot-shaped structure (21).

10. The staple cartridge assembly according to claim 5 or 6, wherein the staple cartridge base (2) is provided with a first slot-shaped structure (21), the staple cartridge assembly is placed into the first slot-shaped structure (21) along a setting direction, and the first section (12a), the second section (12b), and the third section (12c) are sequentially connected along the setting direction.

11. The staple cartridge assembly according to any one of claims 1 to 10, wherein the staple cartridge assembly further comprises a staple cartridge cover (14), the staple cartridge cover (14) is detachably connected with the staple cartridge (11), and the staple cartridge assembly is connected with the staple cartridge base (2) through the staple cartridge cover (14).

12. The staple cartridge assembly according to claim 11, wherein a through hole (141) is provided on the staple cartridge cover (14), the through hole (141) is in communication with the mounting hole (111), and the through hole (141) is configured to allow the limiting portion (122) to pass through.

13. The staple cartridge assembly according to any one of claims 3 to 7, wherein the staple cartridge assembly further comprises a staple cartridge cover (14), the staple cartridge cover (14) is detachably connected with the staple cartridge (11), and the staple cartridge assembly is connected with the staple cartridge base (2) through the staple cartridge cover (14); a through hole (141) is provided on the staple cartridge cover (14), the through hole (141) is in communication with the mounting hole (111), and the through hole (141) is configured to allow the limiting portion (122) to pass through; and the staple cartridge cover (14) tightly presses the fixed end of the elastic piece structure against an end surface at the outer end of the mounting hole (111).

14. The staple cartridge assembly according to any one of claims 1 to 13, wherein first side walls (112) on both sides in a width direction of the staple cartridge (11) are both provided with the mounting holes (111); and/or,
the trigger portion (121) and the limiting portion (122) are both configured as sliding members, the sliding members are slidably mounted on the staple cartridge (11) along a depth direction of the mounting hole (111), the elastic force-transmitting structure (123) is arranged inside the mounting hole (111), and two ends of the elastic force-transmitting structure (123) are respectively in contact with the two sliding members.

15. The staple cartridge assembly according to any one of claims 1 to 14, wherein the trigger portion (121) is provided with a guide surface, and/or the limiting portion (122) is provided with a guide surface.

16. An anastomat, **characterized by** comprising the staple cartridge assembly according to any one of claims 1 to 15.

17. The anastomat according to claim 16, wherein the anastomat further comprises a staple cartridge base (2) and an anvil (3), the staple cartridge base (2) is detachably connected with the staple cartridge assembly, and the anvil (3) is arranged at a top end of the staple cartridge assembly.

18. The anastomat according to claim 16 or 17, wherein a first forming groove is provided on the anvil (3), and a fired staple strikes the first forming groove and undergoes deformation.
